# EUROPEAN PATENT APPLICATION

(11) **EP 4 082 588 A1**
(43) Date of publication of application: **02.11.2022**
(21) Application number: 20907669.4
(22) Date of filing: 23.12.2020
(51) Int. Cl.: A61M 1/28

(54) **PERITONEAL DIALYSIS DEVICE**

(30) Priority: 23.12.2019 KR 20190173473
(71) Applicant: Eoflow Co., Ltd., Bundang-gu, Seongnam-si Gyeonggi-do 13605 (KR)
(72) Inventor: WELSFORD, G. Ian, Stratham, New Hampshire 03885 (US); KIM, Jesse Jaejin, Seongnam-si Gyeonggi-do 13562 (KR); JEON, Yongho, Gwangmyeong-si Gyeonggi-do 14238 (KR)
(74) Representative: Mewburn Ellis LLP
(86) International application number: PCT/KR2020/019030
(87) International publication number: WO 2021/133072

(57) **Abstract**

A peritoneal dialysis device is formed to be connected to the abdominal cavity inside a user's peritoneum, and includes a first connection passage formed to allow the introduction of dialysate that has been injected into the abdominal cavity , an adsorption material unit formed to perform an adsorption process for the dialysate introduced through the first connection passage, an electrolyte control unit formed to control electrolyte supply to the dialysate introduced through the first connection passage, a second connection passage for injecting dialysate treated through the adsorption material unit and the electrolyte control unit back into the abdominal cavity, and a pump unit connected to the second connection passage to control the movement of the dialysate.

## Description

### TECHNICAL FIELD

Embodiments of the present disclosure relate to a peritoneal dialysis device.

### BACKGROUND ART

When a kidney function in body does not operate normally, for example, when there is renal failure, waste products such as uremic in the blood in the body may not be discharged, which may lead to the failure of the maintenance of the body's metabolism, and further to a fatal result.

Various dialysis methods are being implemented for the body with this impaired kidney function.

One of the methods is a blood dialysis method in which a person visits a hospital or clinic several times a week to directly dialysis blood.

In addition, a peritoneum dialysis method is sometimes used to relieve discomfort of visiting a hospital.

The peritoneum dialysis method is convenient because it may be performed at home without visiting a hospital.

However, the peritoneum dialysis has several problems such as inconvenience in replacing dialysate and restrictions in user's movement during dialysis. Therefore, there is a limit in improving convenience and stability of dialysis bodies, for example, patients.

### DESCRIPTION OF EMBODIMENTS

### TECHNICAL PROBLEM

Embodiments of the present disclosure provide a peritoneal dialysis device that improves convenience and stability of dialysis subject.

### TECHNICAL SOLUTION TO PROBLEM

An embodiment of the present disclosure discloses a peritoneal dialysis device formed to be connected to the abdominal cavity inside a user's peritoneum, and including a first connection passage formed to allow the introduction of dialysate that has been injected into the abdominal cavity, an adsorption material unit formed to perform an adsorption process for the dialysate introduced through the first connection passage, an electrolyte control unit formed to control electrolyte supply to the dialysate introduced through the first connection passage, a second connection passage for injecting dialysate treated through the adsorption material unit and the electrolyte control unit back into the abdominal cavity, and a pump unit connected to the second connection passage to control the movement of the dialysate.

In the present embodiment, the peritoneal dialysis device may further include a module unit in which the adsorption material unit and the electrolyte control unit are formed as a single module form.

In the present embodiment, the module unit may be formed to be removable and replaceable from the peritoneal dialysis device.

In the present embodiment, the peritoneal dialysis device may further include a battery that provides power for operation of the peritoneal dialysis device.

In the present embodiment, the peritoneal dialysis device may further include a connection unit, and the connection unit may be formed to be connected to a peritoneal tube extending into the abdominal cavity.

In the present embodiment, the peritoneal dialysis device may further include a connection port formed to connect with a feed tube formed to supply fresh dialysate to the abdominal cavity.

In the present embodiment, the peritoneal dialysis device may be formed to transmit one or more pieces of information by communicating with a terminal provided separately from the peritoneal dialysis device.

In the present embodiment, the peritoneal dialysis device may be attached to an outside of the user's body to have a wearable function.

Other aspects, features and advantages other than those described above will become apparent from the following detailed description of the drawings, claims and disclosure.

### ADVANTAGEOUS EFFECTS OF DISCLOSURE

A peritoneal dialysis device according to the present embodiment may improve convenience and stability of dialysis subject.

### BRIEF DESCRIPTION OF DRAWINGS

FIG. 1 is a view illustrating use of a peritoneal dialysis device according to an embodiment of the present disclosure.
FIG. 2 is a view for explaining the peritoneal dialysis device of FIG. 1.
FIG. 3 is a view for explaining an example of the use of the peritoneal dialysis device of FIG. 1.
FIG. 4 is a view illustrating a part of a modified example of the peritoneal dialysis device of FIG. 1.
FIG. 5 is a view illustrating a part of another modified example of the peritoneal dialysis device of FIG. 1.
FIG. 6 is a view illustrating an alternative embodiment of a pump unit of the peritoneal dialysis device of FIG. 1.
FIG. 7 is a view illustrating another alternative embodiment of a pump unit of the peritoneal dialysis device of FIG. 1.
FIG. 8 is a view illustrating another alternative embodiment of a pump unit of the peritoneal dialysis device of FIG. 1.
FIG. 9 is a cross-sectional view taken along line XIV-XIV of FIG. 8.
FIG. 10 is a view for explaining a peritoneal dialysis device according to another embodiment of the present disclosure.
FIG. 11 is a view for explaining an example of use of the peritoneal dialysis device of FIG. 10.

### MODE OF DISCLOSURE

Since the present disclosure can apply various transformations and can have various embodiments, specific embodiments are illustrated in the drawings and described in detail in the detailed description. Effects and features of the present disclosure, and a method of achieving them will become clear with reference to the embodiments described below in detail in conjunction with the drawings. However, the present disclosure is not limited to the embodiments disclosed below and may be implemented in various forms.

Hereinafter, embodiments of the present disclosure will be described in detail with reference to the accompanying drawings, and when described with reference to the drawings, the same or corresponding components are given the same reference numerals, and overlapping descriptions thereof will be omitted.

In the following embodiments, terms such as first, second, etc. are used for the purpose of distinguishing one component from another, not in a limiting sense.

In the following examples, the singular expression includes the plural expression unless the context clearly dictates otherwise.

In the following embodiments, terms such as include or have means that the features or components described in the specification are present, and the possibility that one or more other features or components will be added is not excluded in advance.

In the drawings, the size of the components may be exaggerated or reduced for convenience of description. For example, since the size and thickness of each component shown in the drawings are arbitrarily indicated for convenience of description, the present invention is not necessarily limited to the illustrated one.

In the following embodiments, the x-axis, the y-axis, and the z-axis are not limited to three axes on a Cartesian coordinate system, and may be interpreted in a broad sense including them. For example, the x-axis, y-axis, and z-axis may be orthogonal to each other, but may refer to different directions that are not orthogonal to each other.

In cases where certain embodiments may be implemented otherwise, a specific process sequence may be performed different from the described sequence. For example, two processes described in succession may be performed substantially simultaneously, or may be performed in an order opposite to the order described.

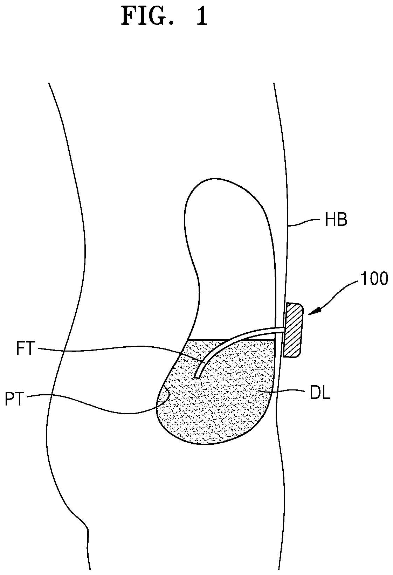

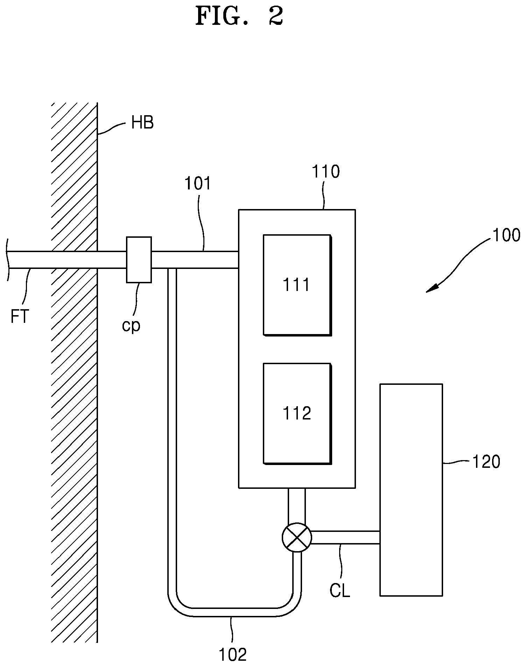

FIG. 1 is a view illustrating use of a peritoneal dialysis device according to an embodiment of the present disclosure, and FIG. 2 is a view for explaining the peritoneal dialysis device of FIG. 1.

Referring to FIG. 1, a peritoneum PT exists inside a user's body HB, and a space, for example, an abdominal cavity, may exist inside the peritoneum PT.

Dialysate DL may be injected into the abdominal cavity, which is the inner space of the peritoneum PT.

Waste products and water in the body may be discharged to the dialysate DL of the abdominal cavity, which is the inner space of the peritoneum PT, through diffusion and osmosis. In addition, after a certain period of time, such as several hours, concentration of the waste product may gradually increase while the dialysate DL stays in the abdominal cavity, and concentration of waste product in blood and the concentration of the waste product in the dialysate DL become the same, and this state is called saturation.

When the waste product concentrations of blood and dialysate DL become the same as the saturation state and waste product no longer moves toward the dialysate, the dialysate DL in the abdominal cavity may be discharged to a peritoneal dialysis device 100.

In addition, fresh dialysate DL may be injected into the abdominal cavity, which is the inner space of the peritoneum PT, through the peritoneal dialysis device 100.

The Waste product of the user's body HB, e.g., uremic that may not be removed from kidneys of patient with chronic renal failure, may be discharged through the dialysate DL of the abdominal cavity, which is the inner space of the peritoneum PT, it uses the "diffusion" process, the waste product in the blood exits into the dialysate DL in the abdominal cavity through a microscopic hole of the peritoneum PT. A rate at which the waste product in the blood exits into the dialysate DL may vary depending on size and type of molecules of the waste product and the concentration of the waste product in the blood.

In addition, in relation to the proper removal of excess water in patients with chronic renal failure who have difficulty excreting water in the user's body HB, such as urine, water in the blood may move to the dialysate DL through osmotic pressure phenomenon. As an optional embodiment, this osmotic pressure phenomenon may be controlled by adjusting the concentration of the dialysate DL.

The peritoneal dialysis device 100 of the present embodiment may be connected to the abdominal cavity of the user's body HB through a peritoneal tube FT.

The peritoneal tube FT may be inserted into the user's body HB before using the peritoneal dialysis device 100. An outside of the user's body HB and the abdominal cavity may be connected through the peritoneal tube FT. For example, the peritoneal tube FT may be a passageway between the outside of the user's body HB and the abdominal cavity.

The peritoneal tube FT may be formed of various materials, for example, may be formed of a flexible and durable material. As a specific example, the peritoneal tube FT may be formed of a silicon material.

The peritoneal dialysis device 100 of the present embodiment will be described in detail with reference to FIG. 2.

The peritoneal dialysis device 100 may be connected to the peritoneal tube FT. For example, the peritoneal dialysis device 100 may include a connection unit cp for connection with the peritoneal tube FT.

As an optional embodiment, the connection unit cp may control the communication between the peritoneal tube FT and the peritoneal dialysis device 100 by including an opening and closing member.

The peritoneal dialysis device 100 may include an adsorption material unit 111 and an electrolyte control unit 112.

The dialysate DL in the abdominal cavity in the peritoneum PT of the user's body HB may flow into the adsorption material unit 111 and the electrolyte control unit 112 from the peritoneal tube FT.

For example, the peritoneal dialysis device 100 may include a first connection passage 101, and the connection unit cp may be formed in an area, as a specific example, an end of the first connection passage 101. Through this, the dialysate DL in the abdominal cavity may be transferred from the peritoneal tube FT to the first connection passage 101, and the dialysate DL may be transferred from the first connection passage 101 to the adsorption material unit 111 and the electrolyte control unit 112.

The adsorption material unit 111 may adsorb the waste product discharged from the user's blood to the dialysate DL in the abdominal cavity. For example, the adsorption material unit 111 may contain various substances so as to adsorb causative substances of the user's uremic disease.

As a specific example, the adsorption material unit 111 may include a ceramic material such as an activated carbon material, an ion exchange resin material, a zeolite material, or alumina.

As an alternative embodiment, the adsorption material unit 111 may include a thin film having a thickness of several nanometers or several tens of nanometers.

For example, the adsorption material unit 111 may include a two-dimensional inorganic compound.

As a specific example, the adsorption material unit 111 may include a layer having a thickness in units of atoms including a transition metal, a carbide or a nitride, and two or more of these.

As an alternative embodiment, the adsorption material unit 111 may include Mxene.

As a specific example, the adsorption material unit 111 may include a nanomaterial composed of a double element of a heavy metal atom and a carbon atom.

In addition, as an example, the adsorption material unit 111 may include a material having a general formula MₐX_{b}, where M is a transition metal, and X is carbon or nitrogen.

As an optional embodiment, the adsorption material unit 111 may include a material of a series having a general formula MₐA_{b}X_{c}, and may include M₍ₙ₊₁₎AXₙ as an example. Here, M is a transition metal, A is a Group 13 or 14 element such as aluminum or silicon, X is carbon or nitrogen, and n may be 1 to 3.

In addition, as an optional embodiment, the adsorption material unit 111 may include a material of a series having a general formula MₐX_{b}T_{c}, and may include M₍ₙ₊₁₎XₙTₘ as an example. Here, M is a transition metal, X is carbon or nitrogen, and T is a functional material, and various elements such as oxygen, fluorine, hydrogen, and chlorine may be selected.

In addition, as an optional embodiment, the adsorption material unit 111 may include a material of a series having a general formula MₐM'_{b}X_{c}, where M is a transition metal, M' is a transition metal different from M, and X is carbon or nitrogen.

The electrolyte control unit 112 may include an electrolyte material, for example, an electrolyte concentrates to control the concentration of electrolyte in the dialysate DL.

As an optional embodiment, the adsorption material unit 111 and the electrolyte control unit 112 may be included in a module unit 110. The module unit 110 may be connected to the first connection passage 101, and the dialysate DL may be transmitted to the module unit 110 through the first connection passage 101.

Dialysate DL in the module unit 110, the adsorption material unit 111 and the electrolyte control unit 112 may be interconnected, for example in direct contact. In addition, as another example, the adsorption material unit 111 may supply the adsorption material to an area where the dialysate DL passes and an electrolyte may be supplied from the electrolyte control unit 112.

As an optional embodiment, the module unit 110 may have a form that may be separated and combined. Through this, the module unit 110 may be replaced in the peritoneal dialysis device 100 and used.

The dialysate DL may be processed while passing through the module unit 110, for example, the waste product may be removed and the electrolyte may be adjusted.

Dialysate DL processed in the module unit 110 may be transmitted back to the abdominal cavity in the user's peritoneum PT through the peritoneal tube FT. For example, the dialysate DL processed in the module unit 110 may be transmitted to the peritoneal tube FT through a second connection passage 102.

As an optional embodiment, the peritoneal dialysis device 100 of the present embodiment may include a pump unit 120.

The pump unit 120 may be connected to a rear end of the adsorption material unit 111 and the electrolyte control unit 112. For example, the pump unit 120 may be connected to a rear end of the module unit 110, and may be connected to one area of the second connection passage 102 as a specific example.

The discharge of dialysate DL from the peritoneal tube FT to the first connection passage 101 may be easily controlled through the control of the pump unit 120. In addition, the dialysate DL processed in the module unit 110 through the control of the pump unit 120 may be controlled to be easily injected into the peritoneal tube FT through the second connection passage 102.

As an optional embodiment, the pump unit 120 and the second connection passage 102 may be connected to each other by a connection unit CL.

As an alternative embodiment, when the dialysate DL is injected into the user's peritoneum PT through the second connection passage 102, it may be injected in a vortex form. For example, one or more flow guide portions, for example, one or more protrusions may be formed on the inner side of the second connection passage 102. In addition, as another example, one or more grooves may be formed inside the second connection passage 102.

In addition, as an optional embodiment, a curved pattern portion, for example, a thread-shaped pattern portion may be formed on the inside of the second connection passage 102. The pattern portion is a region having a different surface shape, and a pattern may be defined as a protruding or concave region.

In addition, as another optional embodiment, the dialysate DL inside the second connection passage 102 may be injected into the peritoneum PT in the vortex form by using the pump unit 120.

In this vortex form, the dialysate DL may be injected into the peritoneum PT to enhance a dialysis effect through the dialysate DL. For example, it may speed up the rate of osmosis between dialysate DL, waste product in the body, water and blood.

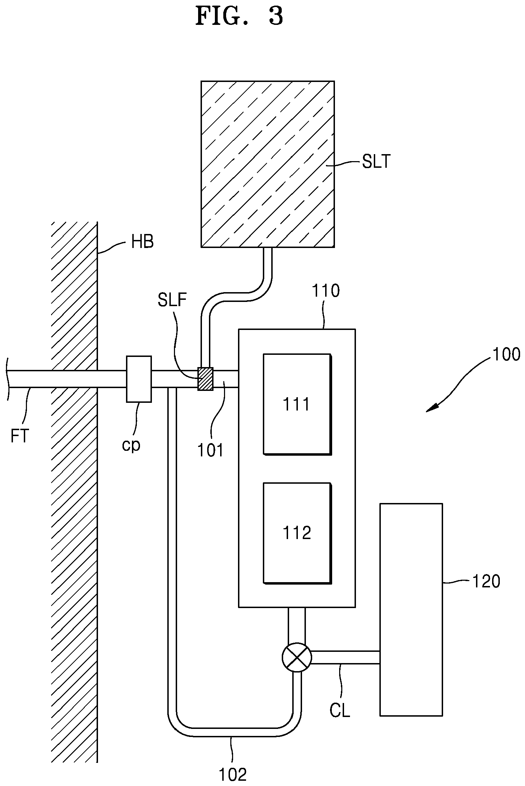

FIG. 3 is a view for explaining an example of the use of the peritoneal dialysis device of FIG. 1.

Referring to FIG. 3, a feed tube SLT may be connected to an area of the peritoneal dialysis device 100 of the present embodiment. For example, the feed tube SLT may be connected to the first connection passage 101 of the peritoneal dialysis device 100 through a connection port SLF.

Also, although not illustrated, the feed tube SLT may be connected to the second connection passage 102 of the peritoneal dialysis device 100.

Dialysate DL may be injected into the abdominal cavity inside the user's peritoneum PT through the feed tube SLT, for example, an initial injection may be performed. After the injection of the dialysate DL, the feed tube SLT is removed, the peritoneal dialysis device 100 is operated, and the dialysate DL treated through the module unit 110 may be injected into the abdominal cavity.

In addition, as an optional embodiment, the feed tube SLT is not removed, and the injection of dialysate may be controlled through the opening and closing operation of the connection port SLF.

The peritoneal dialysis device of the present embodiment may be connected to the abdominal cavity in the user's peritoneum through the peritoneal tube. The dialysate, which remains in the abdominal cavity for a certain period of time and accumulates waste products, may be introduced into the peritoneal dialysis device through the peritoneal tube. The introduced dialysate may be treated while passing through the adsorption material unit and the electrolyte control unit, and the treated dialysate may be injected again into the abdominal cavity in the user's peritoneum through the peritoneal tube.

Through this, the kidney function of users with weakened the kidney function, for example, patients with renal failure may be easily supplemented or replaced.

The user may obtain the effect of kidney function with the peritoneal dialysis device, and it may be maintained for several hours to several days by controlling the amount or characteristics of the adsorption material unit and electrolyte control unit.

In addition, the dialysate may be continuously recirculated through the peritoneal dialysis device, thereby reducing the user's inconvenience and the possibility of infection due to frequent replacement of the dialysate.

As an optional embodiment, the adsorption material unit and the electrolyte control unit may be included in the module unit, and this module unit may be formed to be removable and replaceable in the peritoneal dialysis device. Through this, the module unit that processes the dialysate is replaced after a certain time has elapsed, so that the user's convenience may be improved, and it may be easily used as a wearable device, so that the user's convenience and accessibility may be improved.

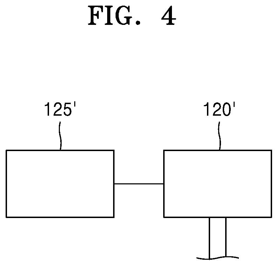

FIG. 4 is a view illustrating a part of a modified example of the peritoneal dialysis device of FIG. 1.

Referring to FIG. 4, the peritoneal dialysis device may include a pump unit 120' and a control unit 125'. The control unit 125' may be connected to the pump unit 120', and may control start, speed, and end of an operation of the pump unit 120'.

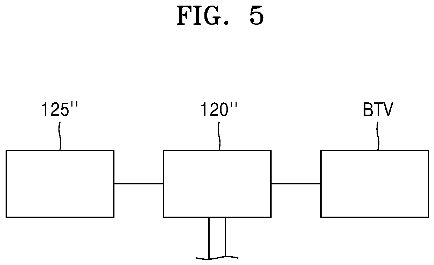

FIG. 5 is a view illustrating a part of another modified example of the peritoneal dialysis device of FIG. 1.

Referring to FIG. 5, the peritoneal dialysis device may include a pump unit 120", a control unit 125" and a battery unit BTV. The control unit 125" may be connected to the pump unit 120", and may control start, speed and end of an operation of the pump unit 120".

The battery unit BTV may be connected to the pump unit 120" to supply power.

In addition, the battery unit BTV may be formed to supply power to at least one region for the entire operation of the peritoneal dialysis device.

As an optional embodiment, the battery unit BTV may have a charging type or a replacement type.

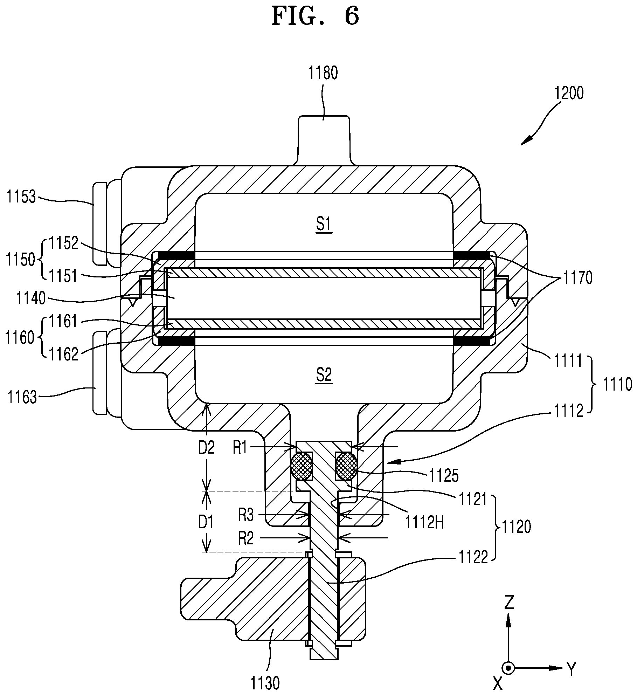

FIG. 6 is a view illustrating an alternative embodiment of a pump unit of the peritoneal dialysis device of FIG. 1.

A pump unit 1200 of the present embodiment may be driven by using a fluid, for example, may include a structure including the fluid in a housing, and may additionally include a membrane.

Referring to FIG. 6, the housing 1110 of the pump unit 1200 includes a shaft hole 1112H provided on one side, and a shaft 1120 having a predetermined length may extend outward the housing 1110 through the shaft hole 1112H. In one embodiment, the shaft hole 1112H may be formed in a protrusion 1112 extending to one side with respect to a body 1111 of the housing 1110, and a diameter of the protrusion 1112 may be formed smaller than a diameter of the body 1111.

A first portion 1121 of the shaft 1120 may be disposed inside the housing 1110, and a second portion 1122 may extend outside of the housing 1110 past the shaft hole 1112H. The shaft 1120 may reciprocate along an up-down direction (z direction) in FIG. 6. When the shaft 1120 reciprocates, the first portion 1121 may linearly reciprocate in an inner space of the housing 1110, for example, an inner space corresponding to the protrusion 1112. Since a diameter R1 of the first portion 1121 of the shaft 1120 is larger than a diameter R3 of the shaft hole 1112H, the first portion 1121 may not fall out of the housing 1110.

As an alternative embodiment, the second portion 1122 of the shaft 1120 may have a smaller diameter R2 than the diameter R3 of the shaft hole 1112H. At this time, the second portion 1122 may be combined with a movement control unit 1130 disposed on the outside of the housing 1110, so that the second portion 1122 is prevented from being pulled out of the shaft hole 1112H.

A sealing material 1125 may be disposed on a side surface of the first portion 1121 of the shaft 1120. The inner space of the housing 1110, for example, a space defined by an inner surface of the housing 1110 and an inner surface of the shaft 1120, is an enclosed space, the fluid exists in the inner space, and the sealing material 1125 may limit fluid leakage through a gap between the housing 1110 and the shaft 1120. For convenience of explanation, FIG. 6 is illustrated with the fluid omitted.

According to an embodiment, as illustrated in FIG. 6, the sealing material 1125 may have an O-ring form and cover the side surface of the first portion 1121, and the leakage of the fluid existing inside the housing 1110 to the outside of the housing 1110 through the shaft hole 1112H may be prevented by the sealing material 1125. The leakage of the fluid may be more effectively limited by making a first distance D1 from the first portion 1121 of the shaft 1120 to the movement control unit 1130 equal to or smaller than an inner length D2 of the protrusion 1112.

The membrane 1140 may be disposed in the inner space of the housing 1110, for example, in the inner space corresponding to the body 1111. The inner space includes a first space S1 and a second space S2 respectively located on both sides of the membrane 1140. In FIG. 2, a space far from the shaft 1120 with respect to the membrane 1140 is the first space S1, and a space adjacent to the shaft 1120 with respect to the membrane 1140 is the second space S2.

The membrane 1140 may have a porous structure in which fluid and ion may move. The membrane 1140 may be, for example, a frit-type membrane prepared by thermally calcining spherical silica. For example, the spherical silica used to form the membrane may have a diameter of about 20 nm to about 500 nm, specifically, may have a diameter of about 30 nm to about 300 nm, and more specifically, may have a diameter of about 40 nm to about 200 nm. When the diameter of the spherical silica satisfies the above-mentioned range, a pressure by a first fluid passing through the membrane 1140, that is, a pressure sufficient to move the shaft 1120 may be generated.

Although it has been described that the membrane 1140 includes the spherical silica, the membrane 1140 is not limited thereto. In another embodiment, any material capable of causing an electrokinetic phenomenon by zeta potential, such as porous silica or porous alumina, may be used for the membrane 1140.

The membrane 1140 may have a thickness of about 20 µm to about 10 mm, specifically, may have a thickness of about 300 µm to about 5 mm, and more specifically, may have a thickness of about 1,000 µm to about 4 mm.

A first electrode body 1150 and a second electrode body 1160 are disposed on both sides of the membrane 1140, respectively. The first electrode body 1150 may include a first porous plate 1151 and a first electrode strip 1152 disposed on a first side of the membrane 1140. The second electrode body 1160 may include a second porous plate 1161 and a second electrode strip 1162 disposed on a second side of the membrane 1140.

The first and second porous plates 1151 and 1161 may be arranged to contact both main surfaces of the membrane 1140, respectively. The first and second porous plates 1151 and 1161 may effectively transport the fluid and the ion through their porous structures. The first and second porous plates 1151 and 1161 may have a structure in which an electrochemical reaction material is formed on a porous base layer. The electrochemical reaction material may be formed by electrodeposition or coating on the porous base layer through methods such as electroless plating, vacuum deposition, coating, and sol-gel process.

The porous base layer may be an insulator. For example, the porous base layer may include at least one selected from a non-conductive ceramic, a non-conductive polymer resin, a non-conductive glass, and a combination thereof.

The non-conductive ceramic may include, for example, at least one selected from a group consisting of rock wool, gypsum, ceramic, cement, and a combination thereof, and specifically, at least one selected from a group consisting of rock wool, gypsum, and a combination thereof, but is not limited thereto.

The non-conductive polymer resin may include, for example, one or more selected from a group consisting of synthetic fiber such as it selected from a group consisting of polypropylene, polyethylene terephthalate, polyacrylonitrile, and a combination thereof; natural fiber such as it selected from a group consisting of wool, cotton, and a combination thereof; sponge; porous material derived from an organism, for example, a bone of the organism; and a combination thereof, but is not limited thereto.

The non-conductive glass may include at least one selected from a group consisting of glass wool, glass frit, porous glass, and a combination thereof, but is not limited thereto.

The porous base layer may have a pore size of about 0.1 µm to about 500 µm, specifically, a pore size of about 5 µm to about 300 µm, and more specifically, a pore size of about 10 µm to about 200 µm. When the pore size of the porous support satisfies the above-mentioned range, it is possible to effectively move the fluid and ion, thereby improving stability, lifespan and efficiency of the pump unit 1200.

The electrochemical reaction material may include a material capable of forming a pair of reactions in which an oxidizing electrode and a reducing electrode exchange cation, for example, hydrogen ion during an electrode reaction of the first and second electrode bodies 1150 and 1160, and at the same time composing a reversible electrochemical reaction. The electrochemical reaction material may include, for example, one or more selected from a group consisting of silver / silver oxide, silver / silver chloride, MnO (OH), polyaniline, polypyrrole, polythiophene, polythionine, or quinone-based polymer, and a combination thereof.

The first and second strips 1152 and 1162 may be disposed on the edges of the first and second porous plates 1151 and 1161, and may be connected to first and second terminals 1153 and 1163 on the outside of the housing 1110. The first and second strips 1152 and 1162 may include a conductive material such as silver, copper, or the like.

The fluid provided in the inner space of the housing 1110 may include the first fluid and a second fluid having different phases. The first fluid may contain a liquid such as water and the second fluid may contain a gas such as air. The first fluid existing in the inner space does not completely fill the inner space. That is, volume of the inner space is larger than volume of the first fluid existing in the inner space. The second fluid exists in a part of the inner space where water does not exist.

A sealing material 1170 is disposed on both sides of a structure of the membrane 1140, the first electrode body 1150 and the second electrode body 1160. The sealing material 1170 may have a ring form having an area corresponding to the edge of the structure described above. The aforementioned fluid, such as the first fluid, moves from the first space S1 to the second space S2 or vice versa along a thickness direction of the membrane 1140 to pass through the membrane 1140, wherein the sealing material 1170 may block a gap between the inner surface of the housing 1110 and the structure to prevent the liquid from moving into the gap.

The fluid may be introduced into the inner space through inlets 1180. In one embodiment, after filling the inner space entirely with the first fluid through the inlets 1180 on both sides, and closing the inlets 1180 after withdrawing a portion of the first fluid through either inlet 1180, the first fluid and the second fluid may exist in the inner space of the housing 1110.

Although not illustrated, the first electrode body 1150 and the second electrode body 1160 may each be connected to a power source through a terminal, and the direction of movement of the liquid such as water may be changed by alternately changing polarities of voltage of the power source.

Also, it is possible to control the movement of the shaft 1120 of the pump unit 1200 through this control. The movement of the shaft 1120 may be connected to the second connection passage 102 of the above-described embodiment to facilitate precise control of the movement of the dialysate DL.

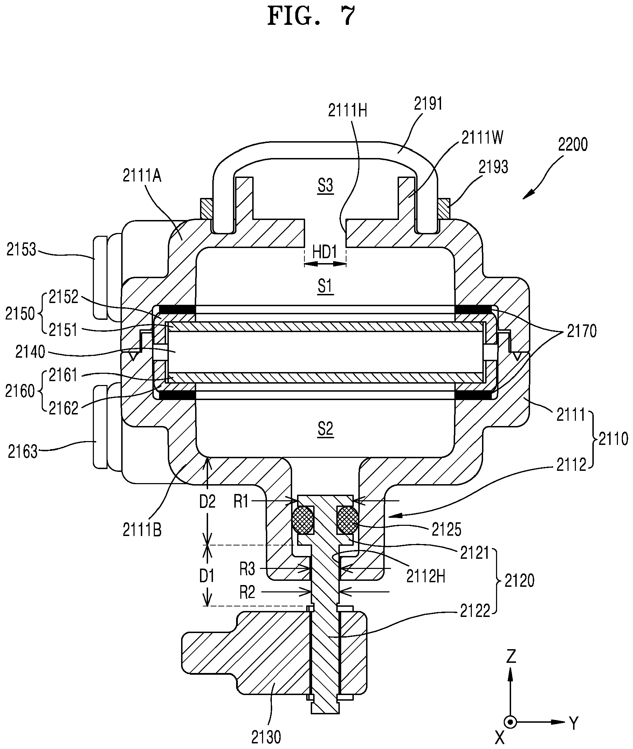

FIG. 7 is a view illustrating another alternative embodiment of a pump unit of the peritoneal dialysis device of FIG. 1.

A pump unit 2200 of the present embodiment may be a type of pump using electric osmotic pressure.

Referring to FIG. 7, a housing 2110 of the pump unit 2200 includes a shaft hole 2112H provided on one side, and a shaft 2120 having a predetermined length may be extended to an outside of the housing 2110 through the shaft hole 2112H.

In one embodiment, the shaft hole 2112H may be formed in a protrusion 2112 extending to one side with respect to a body 2111 of the housing 2110, and a diameter of the protrusion 2112 may be formed smaller than a diameter of the body 2111.

A first portion 2121 of the shaft 2120 is disposed inside the housing 2110, and a second portion 2122 extends to the outside of the housing 2110 through the shaft hole 2112H as described above. The shaft 2120 may reciprocate along an up-down direction (z direction).

The second portion 2122 of the shaft 2120 may have a diameter R2 smaller than a diameter R3 of the shaft hole 2112H, and as an alternative embodiment, the second portion 2122 may be coupled with a movement control unit 2130 disposed outside of the housing 2110.

A sealing material 2125 may be disposed on a side surface of the first portion 2121 of the shaft 2120.

A membrane 2140 may be disposed in an inner space of the housing 2110, for example, in an inner space corresponding to the body 2111. The inner space includes a first space S1 and a second space S2 located on both sides of the membrane 2140, respectively.

In FIG. 7, a space far from the shaft 2120 with respect to the membrane 2140 is the first space S1, and a space adjacent to the shaft 2120 with respect to the membrane 2140 is the second space S2.

The membrane 2140 may have a porous structure in which fluid and ion may move. The membrane 2140 may be, for example, a frit-type membrane prepared by thermally calcining spherical silica. For example, the spherical silica used to form the membrane may have a diameter of about 20 nm to about 500 nm, specifically, may have a diameter of about 30 nm to about 300 nm, and more specifically, may have a diameter of about 40 nm to about 200 nm.

When the diameter of the spherical silica satisfies the above-mentioned range, a pressure by a first fluid passing through the membrane 2140, that is, a pressure sufficient to move the shaft 2120 may be generated.

Although it has been described that the membrane 2140 includes the spherical silica, the membrane 2140 is not limited thereto.

In another embodiment, any material capable of causing an electrokinetic phenomenon by zeta potential, such as porous silica or porous alumina, may be used for the membrane 2140.

The membrane 2140 may have a thickness of about 20 µm to about 10 mm, specifically, may have a thickness of about 300 µm to about 5 mm, and more specifically, may have a thickness of about 1,000 µm to about 4 mm.

A first electrode body 2150 and a second electrode body 2160 are disposed on both sides of the membrane 2140, respectively. The first electrode body 2150 may include a first porous plate 2151 and a first electrode strip 2152 disposed on a first side of the membrane 2140. The second electrode body 2160 may include a second porous plate 2161 and a second electrode strip 2162 disposed on a second side of the membrane 2140.

The first and second porous plates 2151 and 2161 may be arranged to contact both main surfaces of the membrane 2140, respectively. The first and second porous plates 2151 and 2161 may effectively transport fluid and ion through their porous structures.

The first and second porous plates 2151 and 2161 may have a structure in which an electrochemical reaction material is formed on a porous base layer. The electrochemical reaction material may be formed by electrodeposition or coating on the porous base layer through methods such as electroless plating, vacuum deposition, coating, and sol-gel process.

The porous base layer may be an insulator. For example, one or more of the materials described in the embodiment of FIG. 6 may be selectively applied to the porous base layer.

When a pore size of a porous support satisfies the above-mentioned range, it is possible to effectively move the fluid and ion, thereby improving stability, lifespan and efficiency of the pump unit 2200.

The electrochemical reaction material may include a material capable of forming a pair of reactions in which an oxidizing electrode and a reducing electrode exchange cation, for example, hydrogen ion during an electrode reaction of the first and second electrode bodies 2150 and 2160, and at the same time composing a reversible electrochemical reaction.

The electrochemical reaction material may include, for example, one or more selected from a group consisting of silver / silver oxide, silver / silver chloride, MnO (OH), polyaniline, polypyrrole, polythiophene, polythionine, or quinone-based polymer, and a combination thereof.

The first and second strips 2152 and 2162 may be disposed on the edges of the first and second porous plates 2151 and 2161, and may be connected to first and second terminals 2153 and 2163 on the outside of the housing 2110. The first and second strips 2152 and 2162 may include a conductive material such as silver, copper, or the like.

The fluid provided in the inner space of the housing 2110 may include the first fluid and a second fluid having different phases. The first fluid may contain a liquid such as water and the second fluid may contain a gas such as air.

The first fluid existing in the inner space does not completely fill the inner space. That is, volume of the inner space is larger than volume of the first fluid existing in the inner space. The second fluid exists in a part of the inner space where water does not exist.

A sealing material 2170 is disposed on both sides of a structure of the membrane 2140, the first electrode body 2150 and the second electrode body 2160. The sealing material 2170 may have a ring form having an area corresponding to the edge of the structure described above.

The aforementioned fluid, such as the first fluid, moves from the first space S1 to the second space S2 or vice versa along a thickness direction of the membrane 2140 to pass through the membrane 2140, wherein the sealing material 2170 may block a gap between the inner surface of the housing 2110 and the structure to prevent the liquid from moving into the gap.

The fluid may be introduced into the inner space through an inlet 2180. In one embodiment, after filling the inner space with the first fluid entirely through the inlet 2180 on one side and closing the inlet 2180 after withdrawing a portion of the first fluid through the inlet 2180 to the outside, the first fluid and the second fluid may exist in the inner space of the housing 2110.

The motion of the fluid and the movement of the shaft accordingly will be described.

The first electrode body 2150 and the second electrode body 2160 are electrically connected to a power supply unit through the first and second terminals 2153 and 2163, respectively, and polarities of voltage supplied by the power supply unit is alternately changed, so that the direction of movement of the fluid, such as water, may be changed.

In one embodiment, when silver / silver oxide is used as an electrochemical reactant and the first fluid is a solution containing water, when the first electrode body 1150 is the oxidizing electrode and the second electrode body 1160 is the reducing electrode, the cation (e.g., hydrogen ion) generated according to an oxidation reaction in the first electrode body 1150 moves toward the second electrode body 1160 through the membrane 1140 by the voltage difference, at this time, a predetermined pressure may be generated as water (H2O) moves together with the cation.

Through this control, the movement of the shaft 2120 of the pump unit 2200 may be controlled. The movement of the shaft 2120 may be connected to the second connection passage 102 of the above-described embodiment to facilitate precise control of the movement of the dialysate DL.

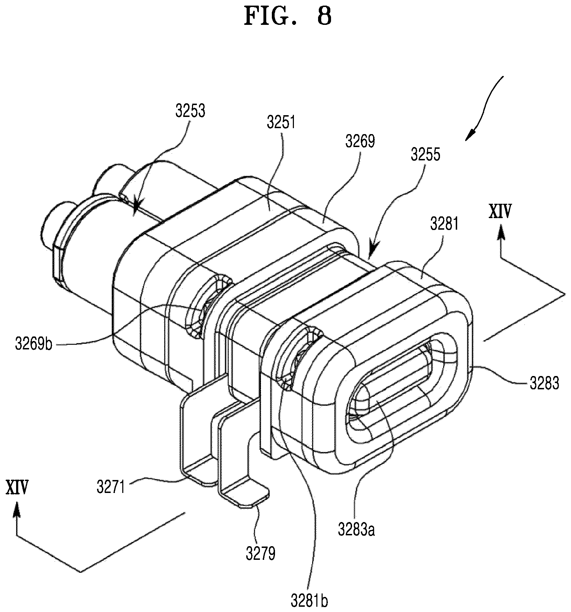

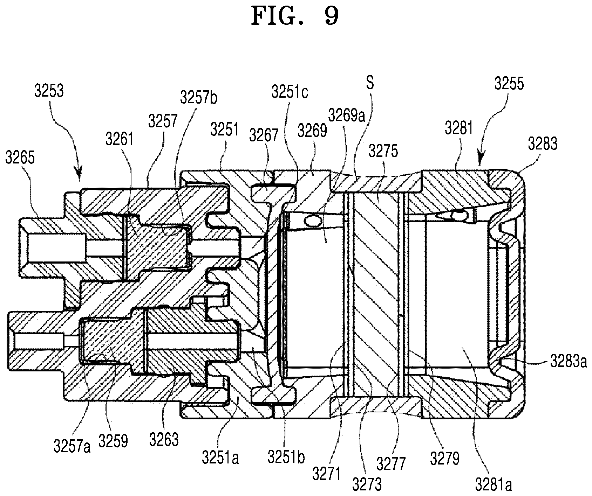

FIG. 8 is a view illustrating another alternative embodiment of a pump unit of the peritoneal dialysis device of FIG. 1. FIG. 9 is a cross-sectional view taken along line XIV-XIV of FIG. 8.

A pump 3200 of the present embodiment may include a connector 3251 and a check valve assembly 3253, and may additionally include a driving unit 3255.

The check valve assembly 3253 may be coupled to one side of the connector 3251, and the driving unit 3255 may be coupled to the other side. The connector 3251 may include a partition wall 3251a that partitions the check valve assembly 3253 and the driving unit 3255. An inlet 3251b and an outlet 3251c may be provided in the partition wall 3251a.

The inlet 3251b and the outlet 3251c are spaced apart from each other and are disposed through the partition wall 3251a. The dialysate DL processed through the above-described module unit 110 flows into the inlet 3251b and then is discharged through the outlet 3251c and may move to the second connection passage 102.

The check valve assembly 3253 may include a valve housing 3257, an inlet check valve 3259, an outlet check valve 3261, a first fixture 3263 and a second fixture 3265.

An inlet extension conduit 3257a and an outlet extension conduit 3257b may be provided in the valve housing 3257. The valve housing 3257 may be coupled to one side of the connector 3251. In addition, the inlet extension conduit 3257a is connected to the inlet 3251b, and the outlet extension conduit 3257b is connected to the outlet 3251c.

The inlet check valve 3259 is placed on the inlet extension conduit 3257a to control a passage direction of the dialysate. The outlet check valve 3261 is disposed on the outlet extension conduit 3257b to control a passage direction of the dialysate.

Flexible, low opening pressure duckbill valves may be used for the inlet check valve 3259 and the outlet check valve 3261. Due to these inlet check valve 3259 and outlet check valve 3261, the transfer efficiency of the fluid is increased compared to the amount of power consumption, so that it may be operated for a long time, and the marketability may be increased.

The first fixture 3263 may be inserted into the inlet extension conduit 3257a to fix the inlet check valve 3259. The second fixture 3265 may be inserted into the outlet extension conduit 3257b to fix the outlet check valve 3261.

The first fixture 3263 and the second fixture 3265 are preferably provided with conduits.

In the embodiment of the present disclosure, an example in which the inlet check valve 3259 and the outlet check valve 3261 are coupled to the valve housing 3257 has been described, but is not limited thereto, and it is also possible that the inlet check valve 3259 and the outlet check valve 3261 are respectively coupled to the inlet 3251b and the outlet 3251c provided in the connector 3251. In this other example, the valve housing 3257 is integrally formed with the connector 3251, so that it may be manufactured with a simpler structure. Another example of the embodiment of the present disclosure may further lower the manufacturing cost by reducing the number of parts, and may be manufactured compactly.

The driving unit 3255 is coupled to one side of the connector 3251. The driving unit 3255 is preferably disposed on the opposite side to which the check valve assembly 3253 is coupled. The driving unit 3255 is preferably arranged in isolation from dialysate passing through the check valve assembly 3253. The driving unit 3255 serves to apply pressure to the dialysate passing through the check valve assembly 3253 through outlet 3251c.

The driving unit 3255 may include a first diaphragm 3267, a first pump housing 3269, a first power supply line 3271, a first electrode 3273, a membrane 3275, a second electrode 3277, a second power supply line 3279, a second pump housing 3281 and a second diaphragm 3283.

The first diaphragm 3267 is coupled to one side of the connector 3251. A space may be provided between the first diaphragm 3267 and the connector 3251. That is, the first diaphragm 3267 is coupled to the connector 3251 by maintaining a constant space in the connector 3251. Therefore, the dialysate in the check valve assembly 3253 does not move to the driving unit 3255 by the first diaphragm 3267 and remains isolated.

A surface of the first diaphragm 3267 may be repeatedly moved in a certain section in an axial direction by the pressure generated by the driving unit 3255. In some cases, the first diaphragm 3267 may be provided with a corrugation portion that allows the surface to move smoothly in the axial direction.

The first diaphragm 3267 described above is coupled to one side of the first pump housing 3269. The first pump housing 3269 may be provided with a perforated space 3269a along the axial direction. Therefore, the first pump housing 3269 may be closed on one side of the space 3269a by the first diaphragm 3267.

The first electrode 3273 is coupled to the other side of the first pump housing 3269 so that the space 3269a formed by the first pump housing 3269 may be closed. And the first pump housing 3269 may accommodate a working fluid such as water in the space 3269a provided therein.

The first pump housing 3269 may be provided with a hole portion 3269b for fluid injection on an outer periphery. This hole portion 3269b may be sealed after the working fluid is injected into the first pump housing 3269. Therefore, the working fluid of the driving unit 3255 may be isolated from the dialysate in the check valve assembly 3253.

The first power supply line 3271 may supply power to the first electrode 3273. The first power supply line 3271 may be disposed along a rim of the first pump housing 3269 and may be fixed in contact with the first electrode 3273. The first power supply line 3271 is preferably disposed between the first pump housing 3269 and the first electrode 3273. However, as another example of the embodiment of the present disclosure, the first power supply line 3271 may be disposed between the first electrode 3273 and the membrane 3275 if only power may be supplied to the first electrode 3273.

The first electrode 3273 may be formed in a plate shape to close the space 3269a of the first pump housing 3269. That is, the first pump housing 3269 may form the space 3269a by the first diaphragm 3267 and the first electrode 3273. In addition, the working fluid such as water is accommodated in the space 3269a of the first pump housing 3269.

The membrane 3275 may be made of a porous material through which the working fluid and ion move. The membrane 3275 is preferably made of a non-conductive material such as ceramic. When the membrane 3275 is made of the non-conductive material, a side reaction does not occur even if the porous membrane 3275 is exposed as the electrochemical reaction material used for the first electrode 3273 and the second electrode 3277 is consumed or desorbed due to the operation of the electric osmosis pump of the present disclosure for a long time. Therefore, unnecessary power consumption due to the side reaction may be prevented. Therefore, the present disclosure has safe driving characteristics and may improve durability.

A flexible material that does not exhibit conductivity such as polymer resin, rubber, urethane or plastic film may be processed into a thin film and used for the membrane 3275.

The second electrode 3277 is disposed on the other side of the membrane 3275. That is, the membrane 3275 is preferably disposed between the first electrode 3273 and the second electrode 3277. The second power supply line 3279 may supply external power to the second electrode 3277. The second power supply line 3279 may be coupled to a rim of the second pump housing 3281. However, as long as the second power supply line 3279 may only supply power to the second electrode 3277, any arrangement is possible.

The second pump housing 3281 has the same or similar shape as the first pump housing 3269. Another space 3281a may be provided through the interior of the second pump housing 3281 in the axial direction. As with the first pump housing 3269, a hole portion 3281b penetrating the inner space 3281a of the second pump housing 3281 may be provided. The hole portion 3281b of the second pump housing 3281 may be sealed by a sealing agent or filled by fusion or the like after injecting the working fluid.

The second diaphragm 3283 may be coupled to one side of the second pump housing 3281 to close the space 3281a provided in the second pump housing 3281.

That is, the second pump housing 3281 may close the space 3281a by the second electrode 3277 and the second diaphragm 3283 made of a plate shape.

In the second diaphragm 3283, a corrugation portion 3283a may be formed on its surface. The corrugation portion 3283a formed in the second diaphragm 3283 may be formed of prominence and depression protruding in the axial direction when viewed from a cross-section. The corrugation portion 3283a of the second diaphragm 3283 may sufficiently move the surface of the second diaphragm 3283 in the axial direction to increase pumping performance.

In the embodiment of the present disclosure, an example in which the corrugation portion 3283a is formed in the second diaphragm 3283 has been described, but in some cases, it is also possible that the corrugation portion is also formed in the first diaphragm 3267. In addition, the corrugation portion that may be formed in the first diaphragm 3267 or the second diaphragm 3283 may reduce energy consumption by maximizing the deformation of the first diaphragm 3267 and the second diaphragm 3283 even with a small amount of energy. That is, there is an advantage that the driving unit 3255 may be driven for a long time even with a small external power source.

The first pump housing 3269, the first power supply line 3271, the first electrode 3273, the membrane 3275, the second electrode 3277, the second power supply line 3279 and the second pump housing 3281 may be sealed from the outside by an encapsulant S. That is, the first power supply line 3271, the first electrode 3273, the membrane 3275, the second electrode 3277 and the second power supply line 3279 are configured to be smaller than the sizes of the first pump housing 3269 and the second pump housing 3281, so that the encapsulant S may be disposed in a peripheral portion (a portion exposed to the outside and forming a groove or space based on a cross-section) between the first pump housing 3269 and the second pump housing 3281 in the assembled state. This encapsulant S may form an airtight layer that maintains airtightness with the outside.

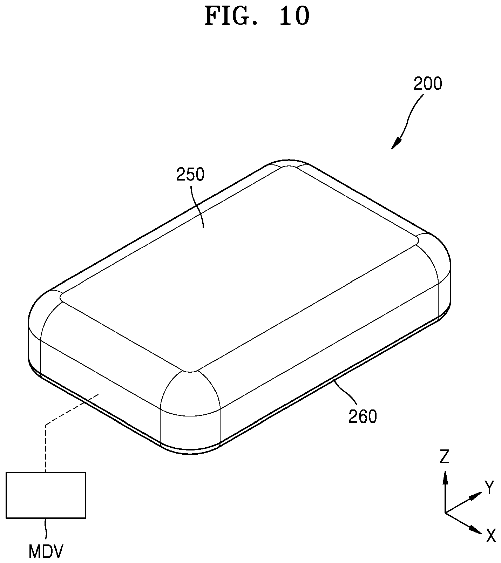

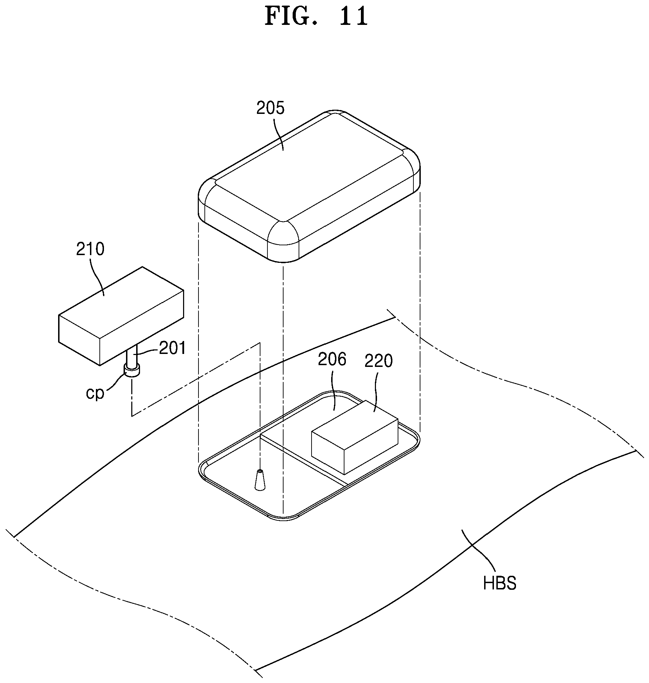

FIG. 10 is a view for explaining a peritoneal dialysis device according to another embodiment of the present disclosure, and FIG. 11 is a view for explaining an example of use of the peritoneal dialysis device of FIG. 10.

Referring to FIGS. 10 and 11, a peritoneal dialysis device 200 may include a cover unit 250 and a base plate 260.

In addition, as an optional embodiment, the peritoneal dialysis device 200 may be connected to an external terminal MDV. The user may start or end an operation of the peritoneal dialysis device 200 through the terminal MDV. In addition, an operating state and performance status of the peritoneal dialysis device 200 may be checked periodically or in real time.

To this end, the terminal MDV may be connected to the peritoneal dialysis device 200 through a wired or wireless communication module, and the peritoneal dialysis device 200 may include such a communication module.

Referring to FIG. 11, the peritoneal dialysis device 200 may include a module unit 210 and a pump unit 220 between the cover unit 250 and the base plate 260.

The module unit 210 may include an adsorption material unit and an electrolyte control unit, which is the same as that described in the above-described embodiment, and a detailed description thereof will be omitted.

The module unit 210 may be a cartridge form, through which it is disposed of after use for a certain period of time, for example, after a period of use such as several days to a week, and a new module unit 210 may be easily replaced.

The use and replacement of the module unit 210 may proceed by connecting and releasing a connection unit cp formed at one end of a first connection passage 201 to a peritoneal tube FT. In addition, the connection with the pump unit 220 may be released and connected.

The base plate 260 includes an opening in one area, through which the peritoneal tube FT connected to the abdominal cavity in the peritoneum of the user's body HB may be placed.

The peritoneal dialysis device 200 of the present embodiment may have a form attached to one area of the user's body HB, and may be used, for example, as a wearable device.

Through this, the user may effectively use the peritoneal dialysis device 200 as the wearable device that may continuously recirculate dialysate.

In addition, when the characteristics of the module unit 210 are reduced due to recirculation, a new module unit 210 may be replaced in a cartridge form, thereby improving user convenience and reducing the cost burden.

As such, the present disclosure has been described with reference to the embodiment shown in the drawings, which is merely exemplary, and those skilled in the art will understand that various modifications and equivalent other embodiments are possible therefrom. Accordingly, the true technical protection scope of the present disclosure should be determined by the technical spirit of the appended claims.

The specific implementations described in the embodiment are only embodiments, and do not limit the scope of the embodiment in any way. For brevity of the specification, descriptions of conventional electronic components, control systems, software, and other functional aspects of the systems may be omitted. In addition, the connection or connection members of lines between the components shown in the drawings illustratively represent functional connections and/or physical or circuit connections, and in an actual device, various functional connections, physical connections that are replaceable or additional may be referred to as connections, or circuit connections. In addition, unless there is a specific reference such as "essential" or "importantly", it may not be a necessary component for the application of this disclosure.

In the specification of embodiments (especially in the claims), the use of the term "above" and similar referential terms may correspond to both the singular and the plural. In addition, when a range is described in the embodiment, each individual value constituting the range is described in the detailed description as including disclosure to which individual values belonging to the range are applied, and if there is no description to the contrary. Finally, the steps constituting the method according to the embodiment may be performed in an appropriate order, unless the order is explicitly stated or there is no description to the contrary. The embodiments are not necessarily limited according to the order of description of the above steps. The use of all examples or exemplary terms (e.g., etc.) in the embodiment is merely for describing the embodiment in detail, and unless it is limited by the claims, the scope of the embodiment is not limited by the examples or exemplary terminology. It is not limited. In addition, those skilled in the art will recognize that various modifications, combinations, and changes may be made in accordance with design conditions and factors within the scope of the appended claims or their equivalents.

## Claims

1. A peritoneal dialysis device formed to be connected to the abdominal cavity inside a user's peritoneum, the peritoneal dialysis device comprising:
a first connection passage formed to allow the introduction of dialysate that has been injected into the abdominal cavity;
an adsorption material unit formed to perform an adsorption process for the dialysate introduced through the first connection passage;
an electrolyte control unit formed to control electrolyte supply to the dialysate introduced through the first connection passage;
a second connection passage for injecting dialysate treated through the adsorption material unit and the electrolyte control unit back into the abdominal cavity; and
a pump unit connected to the second connection passage to control the movement of the dialysate.

2. The peritoneal dialysis device of claim 1, further comprising
a module unit in which the adsorption material unit and the electrolyte control unit are formed as a single module form.

3. The peritoneal dialysis device of claim 2, wherein the module unit is formed to be removable and replaceable from the peritoneal dialysis device.

4. The peritoneal dialysis device of claim 1, further comprising
a battery that provides power for operation of the peritoneal dialysis device.

5. The peritoneal dialysis device of claim 1, wherein
the peritoneal dialysis device further comprises a connection unit, and
the connection unit is formed to be connected to a peritoneal tube extending into the abdominal cavity.

6. The peritoneal dialysis device of claim 1, further comprising
a connection port formed to connect with a feed tube formed to supply fresh dialysate to the abdominal cavity.

7. The peritoneal dialysis device of claim 1, formed to transmit one or more pieces of information by communicating with a terminal provided separately from the peritoneal dialysis device.

8. The peritoneal dialysis device of claim 1, wherein the peritoneal dialysis device is attached to an outside of the user's body to have a wearable function.
